(19) Europäisches Patentamt
European Patent Office
Office européen des brevets

(11) **EP 4 785 974 A1**

(12) **EUROPEAN PATENT APPLICATION**

(43) Date of publication:
05.08.2026 Bulletin 2026/32

(21) Application number: 25155193.3

(22) Date of filing: 31.01.2025

(51) International Patent Classification (IPC):
*A61M 3/02* (2006.01)  *A61M 25/10* (2013.01)

(52) Cooperative Patent Classification (CPC):
A61M 3/0202; A61M 3/0216; A61M 3/022;
A61M 3/0237; A61M 3/0295; A61M 25/10181;
A61M 25/10188; A61M 2205/3334

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Wellspect AB
431 21 Mölndal (SE)

(72) Inventor: D´ESPINDULA, Gabriel
418 75 Göteborg (SE)

(74) Representative: Aldridge, Henry Alexander
Venner Shipley LLP
TIDE Bankside
8 Emerson Street
London SE1 9DU (GB)

(54) **AN IRRIGATION SYSTEM WITH IMPROVED INFLATION CONTROL**

(57) An irrigation system for medical use, such as a rectal irrigation system, and a method for evaluation of such a system, are disclosed. The system comprises a catheter, an irrigation liquid reservoir and an electrically operated diaphragm pump for pumping air into the irrigation liquid reservoir and/or into the catheter. A pressure sensor is associated with an output end of the diaphragm pump and a controller evaluates the operation of the diaphragm pump by: measuring the pressure at two opposite end positions for a diaphragm of the diaphragm pump in at least two different pump cycles; estimating a current effective output volume per pumping cycle based on the measured pressures; comparing this to a previously determined, primary effective output volume; and provide an evaluation of the operation of the diaphragm pump.

Fig. 4

**Description**

TECHNICAL FIELD OF THE INVENTION

The present invention relates to an irrigation system. The irrigation system is particularly intended for rectal, transanal and/or stomal irrigation, and is suitable for self-administration of an irrigation liquid. The invention further relates to a method for evaluation of the operation of a diaphragm pump for pumping air in such an irrigation system.

BACKGROUND OF THE INVENTION

Catheters and catheter systems are used for many types of medical procedures. In some catheter systems, the catheters are provided with retention members, to retain the catheters in place during use when inserted into the patient's/user's body. A common type of retention member for such catheters is an inflatable retention member, such as an inflatable balloon, arranged close to the insertable end of the catheter. A separate lumen is arranged within the catheter for transferring an inflation fluid, such as air, from an external source (e.g. ambient air), through the length of the catheter, and into the inflatable retention member for inflation, and for transferring the inflation fluid back though the length of the catheter during deflation of the inflatable retention member. The inflatable retention member is inflated to a suitable diameter with fluid, such as air, water or saline. The inflation can be made by operation of an electric pump.

The inflatable retention member preferably surrounds the insertion end of the catheter and preferably has a toroidal shape when fully inflated.

In addition to the lumen used for the inflation fluid, a second lumen may be provided to deliver e.g. irrigation fluid to the body cavity, or, alternatively, for drainage of a liquid or the like, such as feces in case of a rectal catheter or urine in case of an indwelling urinary catheter, from the body cavity, etc. For delivery of an irrigation liquid, typically water or saline, to the body cavity it is known to pump the irrigation liquid directly, or to use indirect pumping, where air is pumped into the irrigation liquid reservoir, thereby increasing the pressure in the reservoir and forcing the irrigation liquid to be transferred out of the reservoir. The direct or indirect pumping of irrigation liquid can be made by an electric pump.

However, systems using an inflatable retention member must be used carefully because they can create too much pressure on the body tissue if the retention balloon is overinflated. Accordingly, all such catheter systems must have an indicated maximum volume for the retention balloon that each manufacturer has established as safe. However, this maximum volume can be exceeded by overinflating the inflatable retention member, resulting in potential damage to the soft tissue surrounding the retention member. In particular, overinflation to such a level that the inflatable retention member explodes may be both harmful and painful and may in severe cases even have lethal consequences. However, even without exploding, the inflated retention member can create discomfort, pain or even damage by creating too much pressure on the tissue in vicinity of the inflatable retention member.

Similarly, there is often a need to control the pumping of the irrigation liquid, in order to ensure that a required or preset total volume is pumped, and that the irrigation liquid is transferred at a desired and safe flow rate.

Various attempts have been made over the years to find solutions to better control inflation of such inflatable retention members and/or to better control the pumping of the irrigation liquid. However, most of these proposed solutions are very complex and costly to produce. Further, these known systems are often still not able to provide the user with adequate possibilities to control the inflation, and at the same time ensuring that overinflation is avoided.

For example, US 2016/0193403 by the same applicant discloses an irrigation system in which balloon inflation is controlled in accordance with pre-set inflation levels. However, the inflated size of the balloon may sometime vary from user to user, and also sometimes from time to time for the same user. The inflated size of the balloon may also vary depending on differences in the material of the balloon, due to product variations, tolerances, etc. Further, WO 23/227571 discloses an irrigation system where control of an inflation level is based on pressure measurements. EP 3 338 827 discloses a system and method for controlling inflation of an inflatable balloon where an electrically operated pump is controlled based on both a measured pressure in the balloon and a determined total duration of the pumping.

There is, however, still a need for improvements of the control of inflation of catheters having inflatable retention members in rectal or transanal irrigation systems, but also in other catheter systems having inflatable retention members, such as in urinary catheter systems, in endotracheal intubation systems etc. Similarly, there is a need for improvements in relation to control of the pumping of irrigation system. In particular, there is a need for an irrigation device which can be used safely, easily and conveniently, and with improved controllability, for self-administration of the irrigation liquid, and which also preferably can be produced in a cost-efficient way. It would be desirable to ensure the same or a similar performance in volume delivery regardless of degradations due to previous inflations, material variations, varying body pressures, etc.

SUMMARY OF THE INVENTION

It is therefore an object to provide an irrigation system for medical use, and correspondingly a method for evaluation of the operation of a diaphragm pump in such an irrigation system, which at least alleviates the above-discussed drawbacks of the prior art. In particular, it is an object to provide an irrigation system for use in irrigation procedures, such as in rectal or transanal irrigation.

This and other objects are achieved with an irrigation system according to the appended claims.

According to an aspect, there is provided a method for evaluation of the operation of a diaphragm pump for pumping air in an irrigation system, the method comprising:

operating the diaphragm pump;
measuring pressures at two opposite end positions for a diaphragm of the diaphragm pump in at least two different pump cycles of the diaphragm pump;
estimating a current effective output volume per pumping cycle for the diaphragm pump based on the measured pressures;
comparing the estimated current effective output volume to a previously determined, primary effective output volume; and
provide an evaluation of the operation of the diaphragm pump.

According to another aspect, there is provided an irrigation system for medical use comprising:

a catheter;
an irrigation liquid reservoir;
an electrically operated diaphragm pump for pumping air into the catheter and/or into the irrigation liquid reservoir;
a pressure sensor associated with an output end of the diaphragm pump; and
a controller for evaluation of the operation of the diaphragm pump, the controller being arranged to:

measuring pressures at two opposite end positions for a diaphragm of the diaphragm pump in at least two different pump cycles of the diaphragm pump during operation of the diaphragm pump;
estimating a current effective output volume per pumping cycle for the diaphragm pump based on the measured pressures;
comparing the estimated current effective output volume to a previously determined, primary effective output volume; and
provide an evaluation of the operation of the diaphragm pump.

Preferably, the evaluation is followed by at least one action based on the evaluation, such as issuance of an alarm, control of one or more parameters or processes, or the like.

A diaphragm pump is a well-known pump type which is very affordable and cost-efficient. However, a common problem related to diaphragm pumps is that they are less robust and precise than other types of pumps, and are consequently often unsuitable for use in applications where control of pump volume and/or pump flow with precision is required. A further drawback of diaphragm pumps is that the performance of the pump often varies over time in an unpredictable manner. For example, the volume pumped at each stroke may change over time.

The present invention is based on the understanding that a diaphragm pump may nonetheless be used for applications where there is a need for great precision, such as in irrigation systems for irrigation of a bodily cavity. This is enabled by a novel way of evaluation of the operation of the diaphragm pump. The evaluation may be used for calibration and adjustment of the properties of the diaphragm pump. However, the evaluation may also be used to determine when the performance of the diaphragm pump is too deviant, and in such situations issue an alarm signal, abort operation, or take any other action necessary to prohibit an erroneous pumping.

Thus, with the new evaluation of the diaphragm pump, it becomes possible to use simpler and more-cost-efficient diaphragm pumps in applications where it was previously necessary to use more complex and expensive types of pumps. The herein presented evaluation makes it possible to operate diaphragm pumps with more accuracy, high precision and better control over an extended period of use, and to compensate for any variations that occur over time.

The evaluation enables an estimation of the current effective output volume per pumping cycle, and to compare this with a previously determined primary effective output volume, to check whether the current effective output volume is still within acceptable levels and thereby check if the pump is performing adequately or not. It can also be used for calibration of the pump and a change in the pumping parameters could be made to improve the precision.

The evaluation may be performed before use of the diaphragm pump, in an initialization procedure. Additionally, or alternatively, the evaluation may be performed during operation of the diaphragm pump, e.g. at regular intervals, such as repeatedly after a number of minutes, hours or the like. Alternatively, the evaluation may also be performed continuously.

The pressure sensor may be arranged directly in the diaphragm pump, or be directly coupled to an output of the diaphragm pump. In another embodiment, the pressure sensor may be arranged at a distance from the diaphragm pump, but in the lumen leading from the output of the diaphragm pump ahead of any obstacles that would affect the pressure, such as valves. It is also possible to use more than one pressure sensor, and to arrange the pressure sensors at different locations.

The control of the present invention is also relatively simple to realize, install and operate, thereby making the resulting method/system cost-efficient and user friendly.

A diaphragm pump is of great advantage, in particular in irrigation systems, since it can be operated very easily, which is particularly advantageous for users with reduced dexterity. If the user lacks strength in their hands it may be easier for him/her to operate an electrically operated pump rather than squeezing e.g. a foil-pump. The electrically operated pump can also easily be adjusted and customized for different types of use, for different types of users, etc. It has also been found that diaphragm pumps are cost-efficient, and also relatively simple to analyze, e.g. to determine a correlation between the supplied power and the resulting output air flow.

The method/system may further comprise direct or indirect measurement of an operational speed and/or frequency of the diaphragm pump and estimation of at least one of a pump flow or total pumped volume based on said operational speed and said primary effective output volume. This enables a correct and reliable control of the pump flow and/or pumped volume.

In an embodiment, the operational speed is directly measured by a sensor associated with the diaphragm pump. In another embodiment, the operational speed is indirectly measured by measuring at least one of the operational voltage and current provided to the diaphragm pump. To this end, the irrigation system may further comprise at least one of a current or voltage meter for indirect measurement of an operational speed of the diaphragm pump or a sensor associated with the diaphragm pump for direct measurement of the operation speed of the diaphragm pump.

The pump speed sensor can e.g. be realized as a current sensor, but it could alternatively be any type of encoder (magnetic, light, absolute or incremental), a simple distance sensor to detect the motor's shaft position, or an EMF sensor, sensors embedded in the pump to detect either max, min positions or both, or any type of sensor (capacitive, inductive, light, hall effect) that would provide at least 1 pulse per rotation or an analog signal that could be interpreted as 1 cycle per rotation and/or mechanical revolution.

In an embodiment, the diaphragm pump may, during the step of measuring said pressures, be operated to pump air into a closed reference volume having a known total volume. To this end, the system may further comprise a closed reference volume having a known total volume. In an embodiment, the closed reference volume is at least partly formed by a cavity arranged within a housing which also accommodates the diaphragm pump and fluidly connected to a fluid path extending between the diaphragm pump and the catheter.

The reference volume may be provided in various ways, such as with a separate air tank dedicated to this purpose. However, the reference volume may also be provided by internal volumes already present in the system and with known volumes, such as inner air hoses, tube set when a balloon catheter is new or when a catheter without balloon retention is connected.

In an embodiment, the tube set may be used as a volume reference. In this case, the catheter, if connected to the tube set, is preferably arranged to hold the air and resist inflation during the measurement. This can e.g. be obtained by using a plug instead of a catheter, by using a catheter without inflatable retention element, by using a catheter with a new and previously unexpanded balloon, more resistant to inflation, or the like.

In another embodiment a reference volume in the form of an air tank is provided. The pressure sensor is in this case preferably directly associated to the cavity of the reference volume. One advantage of this system is that it does not rely on connected consumables, such as tube set and catheter, and the system is fully independent during evaluation and calibration.

Even a very limited volume may suffice as a reference volume. For many applications and embodiments, a reference volume as small as only a few ml is enough and provides adequate accuracy. Thus, in an embodiment, the reference volume may have a volume of less than 100 ml, or less than 50 ml, or less than 25 ml, or less than 20 ml, or less than 15 ml, or less than 10 ml. A larger volume would be more precise, but more pump strokes will then be needed, thereby making the evaluation and calibration more time-consuming. If a small volume is used it will take less strokes. An optimal design will consider several factors such as precision of the pressure sensor, speed of the pressure sensor, characteristics of the pump valves, time required to perform this calibration test and so on.

In an embodiment, the estimation of a current effective output volume per pumping cycle for the diaphragm pump comprises determination based on the measured pressures at the end position where the diaphragm is in a fully retracted position and at the end position where the diaphragm is in a fully extended position.

In an exemplary embodiment, a system comprising a diaphragm pump and a tube leading pumped fluid out from the pump, may be considered. The tube may have a length $T_l$, and a tube inner diameter $T_d$. The length and diameter may be used to calculate a total volume $V_T$ of the tube.

The pump chamber has a volume which varies between the two end positions of the diaphragm. The smallest volume $V_{min}$ corresponds to a fully extended diaphragm and the largest volume $V_{max}$ corresponds to a fully retracted diaphragm..

For the evaluation, the pressure is measured at two opposite end positions for the diaphragm of the diaphragm pump in at least two different pump cycles of the diaphragm pump. The two end positions for the diaphragm are when the diaphragm is fully retracted, i.e. where the diaphragm is fully uncompressed and the internal volume is at its maximum, and when the diaphragm is fully extended, i.e. fully pushed into the pump chamber and the internal volume is at its minimum. The equations use Vmax as maximum volume when the diaphragm or piston of the pump is in the "fully uncompressed" position and Vmin is the opposite, when the piston is all the way up and the chamber has the minimum volume.

For example, in a first stroke the pressure $p_1$ may be measured when the diaphragm is fully retracted and a pressure $p_2$ when the diaphragm is fully extended, and correspondingly in a second stroke, the pressure $p_3$ may be measured when the diaphragm is fully retracted and pressure $p_4$ when the diaphragm is fully extended.

The total pressure-volume product of the tube and chamber at pressure $p_1$, and with an atmospheric pressure $p_{atm}$, may be formulated as:

$$(V_T+V_{max}*p_{atm}/p_1)*p_1$$

Correspondingly, the total pressure-volume product of the tube and chamber at pressure $p_2$, and with an atmospheric pressure $p_{atm}$, may be formulated as:

$$(V_T+V_{min})*p_2$$

The following relation between the pressure-volume products are present:

$$(V_T+V_{max}*p_{atm}/p_1)*p_1 = (V_T+V_{min})*p_2$$

This equation can be rewritten to a general form of ax + b = y, where a would correspond to $V_{max}$ and b to $V_{min}$ in the following way:

$$(p_{atm}/p_2)*V_{max}+(V_T*(p_1-p_2)/p_2)=V_{min}$$

Based on this equation, and based on the measured end values of the pressure at two or more consecutive strokes, it is possible to determine $V_{max}$ and $V_{min}$ by the intersections of the lines. For example, $V_{max}$ may be determined as:

$$V_{max}=((V_T*(p_3-p_4)/p_4)-(V_T*(p_1-p_2)/p_2))/((p_{atm}/p_2)-(p_{atm}/p_4))$$

After simplifications it has been found that Vmax and Vmin could be determined, based on the measured pressures, in the following way:

$$V_{max}=(V_T*(p_2*p_3-p_1*p_4))/(p_{atm}*(p_4-p_2))$$

$$V_{min}=(V_T*(p_3-p_1-p_4+p_2))/(p_4-p_2)$$

The estimation of a current effective output volume per pumping cycle for the diaphragm pump may comprise determination based on the measured pressures of a maximum volume for an end position where the diaphragm is in a fully retracted position and a minimum volume for an end position where the diaphragm is in a fully extended position. Based on the equations above, a current effective output volume $V_E$ per pumping cycle for the diaphragm pump, based on the measured pressures, may e.g. be calculated as:

$$V_E = V_{max} * P_{in} / P_{out} - V_{min}$$

where $P_{in}$ may be e.g. $P_1$ or $P_3$ and $P_{out}$ may be e.g. $P_2$ or $P_4$.

The evaluation, and a possible calibration based on the evaluation, may be used in various ways, such as for a change in parameters to improve the precision, but can also simply check if the estimated volumes are still within acceptable levels. In this case the estimated effective volume could be used to just check if the pump is good enough or not.

In an embodiment, the provision of the evaluation of the operation of the diaphragm pump comprises determining whether a difference between the current effective output volume and the primary effective output volume exceeds a first threshold and, if this is the case, assigning a new value to the primary effective output based on the estimation of the current effective output volume. Hereby, the performance of the diaphragm pump may continue and still be adequately controllable. A smaller or higher effective output volume may be compensated by adjusting the speed of the pump or how long the pump is operated in order to still obtain e.g. a desired total pumped volume, a desired pumped flow rate, etc.

In an embodiment, the provision of the evaluation of the operation of the diaphragm pump comprises determining whether a difference between the current effective output volume and the primary effective output volume exceeds a second threshold and, if this is the case, adjust at least one operational parameter of the diaphragm pump to decrease the difference between the current effective output volume and the primary effective output volume. The adjustment may involve adjustment of the diaphragm in the diaphragm pump, or the operation of the pump, such as pump speed, etc.

In an embodiment, the provision of the evaluation of the operation of the diaphragm pump comprises determining of whether a difference between the current effective output volume and the primary effective output volume exceeds a third threshold and, if this is the case, issue an alarm signal and/or make the diaphragm pump inoperable. The alarm signal may be directed to the operator and may e.g. be an audible alarm signal, such as a beeping sound, a visible alarm signal, such as a flashing light, or the like. The operator may then carry on with the operation of the diaphragm pump, but with greater precaution, or may decide to abort the operation. The alarm signal may also be an electric signal, a radio signal, or the like, sent to an internal or external controller. The internal or external controller may then decide whether to take any actions based on the alarm signal.

The diaphragm pump may be used to pump both liquids and gases. In the present context, the diaphragm pump is preferably arranged to pump a gas, and most preferably air.

The diaphragm pump may be used for various types of applications, but is particularly advantageous for use in medical irrigation systems, and specifically for medical irrigation systems for irrigation of a bodily cavity, such as the rectum or anal canal. The diaphragm pump may be used for various functions. The diaphragm pump may e.gt. be used for pumping a fluid, and in particular air, to an inflatable retention member, such as an inflatable balloon arranged in the vicinity of an insertable tip of a probe or catheter insertable into the bodily cavity. Additionally, or alternatively, the diaphragm pump may be used to pump irrigation liquid to the irrigation site, i.e. into the bodily cavity. To this end, the diaphragm pump may be used for indirect pumping of the irrigation liquid, by pumping a fluid, such as air, into a reservoir accommodating the irrigation liquid, thereby by indirect action pressing the irrigation liquid out from the reservoir.

In an embodiment, the catheter has an inflatable retention member and wherein the electrically operated diaphragm pump is arranged to pump air into the catheter for inflation of the inflatable retention member. Catheters with inflatable retention members, such as an inflatable balloon, may be used in many medical procedures where there is a need to retain the catheter in an inserted position during use. For example, such catheters are commonly used when the catheter is intended for insertion into the rectum, so-called rectal catheters, for example for use in anal irrigation systems, and for indwelling urinary catheters, for draining of urine from the bladder.

Additionally, or alternatively, the electrically operated diaphragm pump may be arranged to pump air into the irrigation liquid reservoir for indirect pumping of irrigation liquid from the irrigation liquid reservoir to the catheter.

The catheter is preferably a rectal catheter or probe, and wherein the catheter system is a rectal, anal, stomal or transanal irrigation system.

The system and method of the present invention is particularly useful for use in rectal or anal irrigation system, as well as in stomal irrigation. However, the method/system of the present invention is also useable in other catheter systems having e.g. inflatable retention members, such as in urinary catheter systems, in endotracheal intubation systems etc.

In rectal or anal irrigation, an irrigation liquid is introduced into the rectum and lower intestine of a patient in order to induce bowel movement. The need for such a procedure typically arises in patients suffering from certain physical ailments in which voluntary bowel control is impaired or when the bowel needs to be cleaned before e.g. a coloscopy or a surgical operation. To this end, irrigation systems may be used e.g. by people suffering from spinal cord injuries, spina bifida or multiple sclerosis. For such users, irrigation may improve quality of life by preventing constipation, reducing time spent for bowel emptying procedures, reducing fecal incontinence, and by increasing independency and quality of life in general.

The evaluation and calibration as discussed in the foregoing are particularly useful in irrigation systems where the flow rate for the irrigation liquid and/or the size of an inflatable retention element are controlled based on calculations relating to the volume of air pumped per stroke. Such irrigation systems are e.g. known from European patent application no. 23202424.0 and European patent application no. 23202446.3, both by the same applicant and both hereby in their entirety incorporated by reference. Since the effective output volume may change over time, due to variations in the performance of the diaphragm pump, and also through leakage through valves, etc, the use of the present evaluation is highly useful for a calibration of the pump, or a measurement of the pump and subsequent adjustment of the calculations.

More specifically, European patent application no. 23202424.0 is directed to a method and system for improved flow control of an irrigation liquid being pumped into the receiving site, such as the anal canal or rectum in case of a rectal or transanal irrigation system. The system comprises: a reservoir for an irrigating liquid; a pressure sensor for measuring the pressure in the reservoir; and a catheter for arrangement in a user; tubing providing a fluid communication path between said reservoir and said catheter. An electrically operated pump is provided for pumping air into the reservoir, thereby to indirectly pump irrigation liquid from the reservoir to the catheter through said tubing. Further, an electrically operable valve is operable to continuously control the degree of openness of said fluid communication path between a fully closed state and a fully opened state. A sensor is provided for estimation of power consumption or speed of the electrically operated pump and a controller is arranged to estimate an actual flow rate of irrigation liquid from the reservoir to the catheter based on said estimation of the power consumption and/or speed, to obtain a desired flow rate from a user interface, and to continuously regulate the electrically operable valve based on the estimated actual flow rate, thereby adjusting the actual flow rate to said desired flow rate. Hereby, the actual flow rate can be estimated without any need for flow meters and the like. The above-discussed evaluation and calibration enables use of such a control together with a diaphragm pump with increased precision and accuracy.

European patent application no. 23202446.3 is directed to a method and system for improved inflation control of an inflatable retention element, e.g. arranged on an insertable end of a rectal catheter or probe in case of a rectal or transanal irrigation system. The irrigation system comprises: a catheter having an inflatable retention member; an electrically operated pump for pumping a fluid into the catheter for inflation of the inflatable retention member; and an electrically operated valve for deflation of the inflatable retention member. A controller is provided for controlling the electrically operated pump and the electrically operated valve, the controller arranged to determine an inflation flow rate into the inflatable retention member from the electrically operated pump, and a deflation flow rate out from the inflatable retention member through the electrically operated valve, to continuously determine an inflation fluid volume based on the inflation flow rate and a deflation fluid volume based on the deflation flow rate, to continuously determine a present fluid volume in the inflatable retention member as the inflation fluid volume reduced by the deflation fluid volume, and to prohibit operation of the electrically operated pump when the present fluid volume is determined to have reached a volume safety threshold. Also here, the above-discussed evaluation and calibration enables use of such a control together with a diaphragm pump with increased precision and accuracy.

The irrigation system of the present invention is preferably portable, of limited size and relatively simple to use and control, also for user's having reduced dexterity. This makes it very well suited for self-administration, i.e. when irrigation is performed outside medical attendance premises, such as in the patient's home, and is performed by the patient himself. Further, portability of the irrigation system is of advantage for disabled persons who are not hospitalized or bed-ridden if they are to live as normal a life as possible. This is particularly important if they travel away from their home, for instance, to someone else's home, or if they stay in a hotel. In this situation, they need to be able to deal with their bowel function easily.

Preferably, all components of the irrigation system are individually exchangeable, so that e.g. the probe/catheter can be exchanged frequently, and typically be used only once, whereas other parts of the system, such as the control unit, the electrical system and the irrigation liquid reservoir can be used for months or even years.

The irrigation system of the present invention comprises relatively few and uncomplicated components, which may be reused for a long time, which makes the irrigation system relatively easy and cost-efficient to produce. Further, the irrigation system lends itself well for automated or semi-automated manufacturing.

These and other aspects of the invention will be apparent from and elucidated with reference to the embodiments described hereinafter.

BRIEF DESCRIPTION OF THE DRAWINGS

For exemplifying purposes, the invention will be described in closer detail in the following, with reference to embodiments thereof illustrated in the attached drawings, wherein:

Fig. 1 illustrates an irrigation system in accordance with an embodiment of the present disclosure;
Fig. 2 schematically illustrates a side view of a container useable in embodiments of the present disclosure;
Fig. 3 schematically illustrates a side view of a container useable in another embodiment of the present disclosure;
Fig. 4 is a schematic block diagram of an irrigation system in accordance with an embodiment of the present disclosure;
Fig. 5 is a schematic block diagram illustrating the parts of the irrigation system in Fig. 4 useable for inflation and deflation control in more detail;
Fig. 6. is a flow chart illustrating a method for controlling inflation in, accordance with an embodiment of the present disclosure;
Fig. 7 is a schematic block diagram illustrating the parts of the irrigation system in Fig. 4, useable for irrigation fluid flow control in more detail; and
Fig. 8 is a schematic block diagram illustrating a possible realization of the flow control system of Fig. 7 in more detail.

DETAILED DESCRIPTION OF CURRENTLY PREFERRED EMBODIMENTS

The irrigation system of the present disclosure is particularly useful for rectal and transanal irrigation. However, the irrigation system may also be used in other types of medical irrigation procedures. The following discussion is, in particular, concerned with the preferred field of use, rectal and transanal irrigation systems, even though the disclosure is not limited to this particular type of irrigation systems.

It is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, e.g. the length and width of the container, etc.

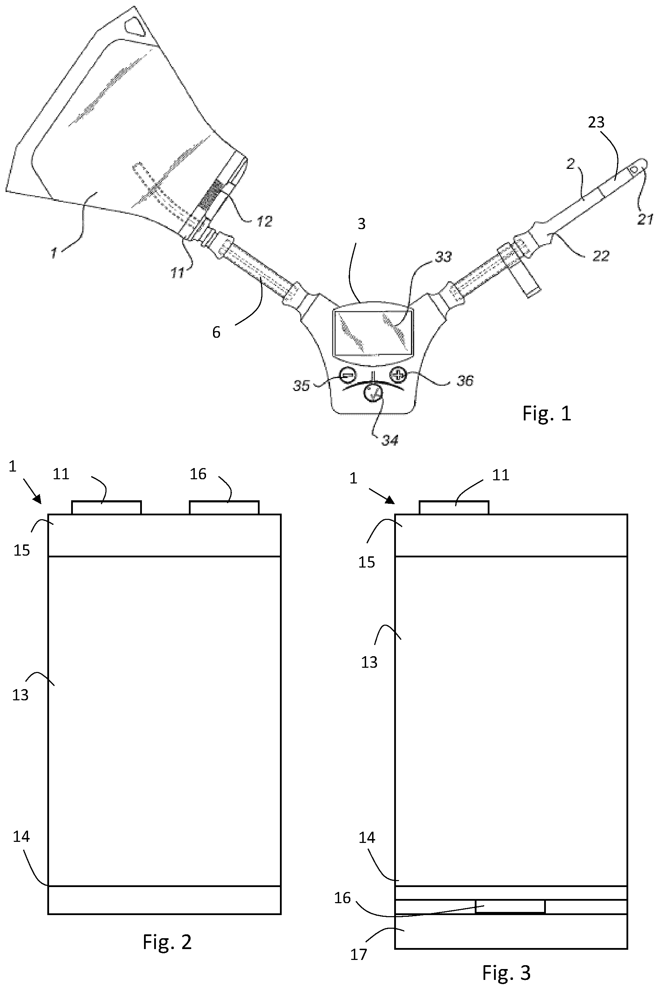

Fig. 1 discloses an irrigation system according to a first exemplary embodiment, comprising a container 1 for an irrigation liquid, a catheter or probe 2 for arrangement in a user, and a control unit 3.

The container may be realized in various ways. For example, the container may be formed by a rigid, semi-rigid or flexible material. In case a semi-rigid or flexible material is used, the container may be collapsible or foldable, to make the irrigation system more compact prior to use. The container is preferably provided with an opening, closed by a closure 11, for filling of the reservoir. Tubing connecting the container to the rest of the irrigation system may be provided through the closure 11, or through other access points on the container.

In order to render the irrigation system as portable as possible, the container preferably has a capacity of less than 5 liters, more preferred less than 3 liters and most preferred less than 2 liters. If, however, the system is to be used for repeated irrigation, a larger capacity container may be necessary.

The container may comprise an overpressure release valve, to release pressure over a predetermined maximum pressure to be allowed. Further, the container may also, optionally, comprise a filter 12, such as a hydrophobic filter, which is impermeable to the irrigation liquid, but which allows air to enter the reservoir but not escape the container. Such a filter ensures that the container maintains its shape when irrigation liquid is being pumped out from the container.

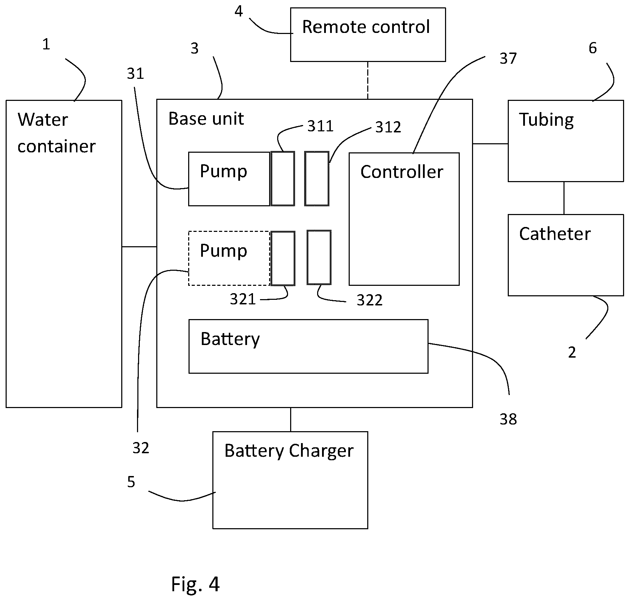

A pump 31, best seen in Fig. 4, may be used to pump air into the container for, indirectly, pumping liquid out of the container. Alternatively, the pump may directly pump liquid out of the container.

The catheter/probe 2 is provided with a retention member, such as an inflatable balloon 23, in the vicinity of an insertable end 21, for fixing the catheter in a body cavity.

The pump 31 used for indirect or direct pumping of irrigation liquid from the water container may also be used for pumping air or other fluids into the inflatable retention element. The pump 31 may e.g. be arranged to pump air, and valves may be used to direct the pumped air into the water container and the inflatable retention member, respectively. However, alternatively, a second pump 32 may be provided to pump air or other fluids into the inflatable retention member.

In a preferred embodiment, the pump 31 is arranged for indirect pumping of the irrigation liquid. Hereby, the pump pumps a fluid, preferably a gas such as air, through a conduit to the reservoir. Thereby, pressure increases in the reservoir to pump irrigation liquid through another conduit to the control unit. This conduit preferably passes through an electrically operable valve and optionally a flow sensor (not shown) and continues to the probe, for dispensing the irrigation liquid to the user. The valve is preferably connected to the controller 37 (see Fig. 4), so that the controller may control the degree of openness of the valve. In case a flow sensor is provided, the input from the flow sensor may be used by the controller to regulate the valve.

The valve may be an on/off valve, arranged only to assume a fully opened or fully closed state. However, the valve may also provide intermediate positions, and may e.g. be gradually controllable between these end states. Such an electrically operable valve can be realized in many ways, as is per se well-known in the art. For example, the electrically operable valve may be a clamp or pinch valve, providing a controllable clamping/pinching action on a tube leading between the electrical pump and the probe. For example, the valve may be of the type disclosed in US 2006/0114148 by the same applicant, said document hereby being incorporated in its entirety by reference.

At least one of the one or more pumps are electrically operated pump, and in particular diaphragm pump(s).

To each diaphragm pump 31, 32 is associated a pressure sensor 311, 321, for measuring the pressure at two opposite end positions for a diaphragm of the diaphragm pump in at least two different pump cycles of the diaphragm pump. Based on the measured pressures a current effective output volume per pumping cycle for the diaphragm pump can be estimated, and compared to a previously determined, primary effective output volume, in order to provide an evaluation of the operation of the diaphragm pump. The evaluation may be used for calibration and adjustment of the properties of the diaphragm pump. However, the evaluation may also be used to determine when the performance of the diaphragm pump is too deviant, and in such situations issue an alarm signal, abort operation, or take any other action necessary to prohibit an erroneous pumping.

The evaluation may be used to check whether the current effective output volume is still within acceptable levels and thereby check if the pump is performing adequately or not. It can also be used for calibration of the pump and a change in the pumping parameters could be made to improve the precision.

The evaluation may be performed before use of the diaphragm pump, in an initialization procedure. Additionally, or alternatively, the evaluation may be performed during operation of the diaphragm pump, e.g. at regular intervals, such as repeatedly after a number of minutes, hours or the like. Alternatively, the evaluation may also be performed continuously.

The pressure sensor(s) 311, 321 may be arranged directly in the diaphragm pump, or be directly coupled to an output of the diaphragm pump. In another embodiment, the pressure sensor may be arranged at a distance from the diaphragm pump, but in the lumen leading from the output of the diaphragm pump ahead of any obstacles that would affect the pressure, such as valves. It is also possible to use more than one pressure sensor, and to arrange the pressure sensors at different locations.

In an embodiment, the diaphragm pump may, during the step of measuring said pressure , be operated to pump air into a closed reference volume 312, 322 having a known total volume. To this end, the system may further comprise a closed reference volume having a known total volume. In an embodiment, the closed reference volume is at least partly formed by a cavity arranged within a housing, which also accommodates the diaphragm pump, and fluidly connected to a fluid path extending between the diaphragm pump and the catheter.

The reference volume may be provided in various ways, such as with a separate, air tank 312, 322 dedicated to this purpose. In this case, the pressure sensor may be directly associated to the cavity of the reference volume. However, the reference volume may also be provided by internal volumes already present in the system and with known volumes, such as inner air hoses, tube set when a balloon catheter is new or when a catheter without inflation retention member is connected.

Only a very limited volume would suffice as a reference volume. For many applications and embodiments, a reference volume as small as only a few ml is enough and provides adequate accuracy. Thus, in an embodiment, the reference volume may have a volume of less than 100 ml, or less than 50 ml, or less than 25 ml, or less than 20 ml, or less than 15 ml, or less than 10 ml. A larger volume would be more precise, but mor pump strokes will then be needed, thereby making the evaluation and calibration more complicated and time-consuming. If a small volume is used it will take less strokes., An optimal design will consider several factors as precision of the pressure sensor, speed of the pressure sensor, characteristics of the pump valves, time required to perform this calibration test and so on.

The estimating of a current effective output volume per pumping cycle for the diaphragm pump may comprise determination based on the measured pressures at the end position. In one end position the diaphragm is in a fully retracted position and at another end position the diaphragm is in a fully extended position. Thus, in the pump cycle, the end positions represent a maximum volume at one end position, where the diaphragm is in a fully retracted position, and a minimum volume at one end position, where the diaphragm is in a fully extended position.

In an exemplary embodiment, a system comprising a diaphragm pump and a tube leading pumped fluid out from the pump may be considered. The tube may have a length $T_l$, e.g. in the range of 0.5-2 m, such as 1.2 m, and a tube inner diameter Ta, e.g. in the range of 2-7 mm, such as 4.2 mm. The length and diameter may be used to calculate a total volume $V_T$ of the tube, which may e.g. be in the range of 5-20 ml, such as 11 ml.

The pump chamber has a volume which varies between the two end positions of the diaphragm. The smallest volume $V_{min}$ corresponds to a fully extended diaphragm and the largest volume $V_{max}$ corresponds to a fully retracted diaphragm. In an exemplary diaphragm pump, $V_{min}$ could e.g. be in the range 0.05-0.30 ml, and $V_{max}$ could e.g. be in the range 0.30-1.00 ml.

For the evaluation, the pressure is measured at two opposite end positions for the diaphragm of the diaphragm pump in at least two different pump cycles of the diaphragm pump. The two end positions for the diaphragm are when the diaphragm is fully retracted, i.e. where the diaphragm is fully uncompressed, and the internal volume is at its maximum, and when the diaphragm is fully extended, i.e. fully pushed into the pump chamber, and the internal volume is at its minimum. The equations use $V_{max}$ as maximum volume (when the diaphragm or piston of the pump is in the "fully uncompressed" position and $V_{min}$ is the opposite, when the piston is all the way up and the chamber has the minimum volume.

For example, in a first stroke the pressure $p_1$ may be measured when the diaphragm is fully retracted and a pressure $p_2$ when the diaphragm is fully extended, and correspondingly in a second stroke, the pressure $p_3$ may be measured when the diaphragm is fully retracted and pressure $p_4$ when the diaphragm is fully extended.

The total pressure-volume product of the tube and chamber at pressure $p_1$, and with an atmospheric pressure $p_{atm}$, may be formulated as:

$$(V_T + V_{max} * p_{atm}/p_1) * p_1$$

Correspondingly, the total pressure-volume product of the tube and chamber at pressure $p_2$, and with an atmospheric pressure $p_{atm}$, may be formulated as:

$$(V_T + V_{min}) * p2$$

The following relation between the pressure-volume products are present:

$$(V_T+V_{max}*p_{atm}/p_1)*p_1 = (V_T+V_{min})*p_2$$

This equation can be rewritten to a general form of ax + b = y in the following way:

$$(p_{atm}/p_2)*V_{max}+(V_T*(p_1-p_2)/p_2)=V_{min}$$

Based on this equation, and based on the measured end values of the pressure at two or more consecutive strokes, it is possible to determine $V_{max}$ and $V_{min}$ by the intersections of the lines. For example, $V_{max}$ may be determined as:

$$V_{max}=((V_T*(p_3-p_4)/p_4)-(V_T*(p_1-p_2)/p_2))/((p_{atm}/p_2)-(p_{atm}/p_4))$$

After simplifications it has been found that $V_{max}$ and $V_{min}$ could be determined, based on the measured pressures, in the following way:

$$V_{max}=(V_T*(p_2*p_3-p_1*p_4))/(p_{atm}*(p_4-p_2))$$

$$V_{min}=(V_T*(p_3-p_1-p_4+p_2))/(p_4-p_2)$$

The estimating of a current effective output volume per pumping cycle for the diaphragm pump may comprise determination based on the measured pressures of a maximum volume for an end position where the diaphragm is in a fully retracted position and a minimum volume for an end position where the diaphragm is in a fully extended position. Based on the equations above, a current effective output volume per pumping cycle for the diaphragm pump, based on the measured pressures, may e.g. be calculated as:

$$V_E = P_{in} * V_{max} / \ P_{out} - V_{min}$$

where $P_{in}$ may be e.g. $P_1$ or $P_3$ and $P_{out}$ may be e.g. $P_2$ or $P_4$.

The evaluation, and a possible calibration based on the evaluation, may be used in various ways, such as for a change in parameters to improve the precision, but can also simply check if the estimated volumes are still within acceptable levels. In this case the estimated effective volume could be used to check if the pump is good enough or not.

In an embodiment, the provision of the evaluation of the operation of the diaphragm pump comprises determining of whether a difference between the current effective output volume and the primary effective output volume exceeds a first threshold, and if this is the case assigning a new value to the primary effective output based on the estimation of the current effective output volume. Hereby, the performance of the diaphragm pump may continue and still be adequately controllable. A smaller or higher effective output volume may be compensated by adjusting the speed of the pump or how long the pump is operated in order to still obtain e.g. a desired total pumped volume, a desired pumped flow rate, etc.

In an embodiment, the provision of the evaluation of the operation of the diaphragm pump comprises determining of whether a difference between the current effective output volume and the primary effective output volume exceeds a second threshold, and if this is the case adjust at least one operational parameter of the diaphragm pump to decrease the difference between the current effective output volume and the primary effective output volume. The adjustment may involve adjustment of the diaphragm in the diaphragm pump, or the operation of the pump, such as pump speed, etc.

In an embodiment, the provision of the evaluation of the operation of the diaphragm pump comprises determining of whether a difference between the current effective output volume and the primary effective output volume exceeds a third threshold, and if this is the case, issue an alarm signal and/or make the diaphragm pump inoperable. The alarm signal may be directed to the operator and may e.g. be an audible alarm signal, such as a beeping sound, a visible alarm signal, such as a flashing light, or the like. The operator may then carry on with the operation of the diaphragm pump, but with greater precaution, or may decide to abort the operation. The alarm signal may also be an electric signal, a radio signal, or the like, sent to an internal or external controller. The internal or external controller may then decide whether to take any actions based on the alarm signal.

The reservoir/container 1 may be realized in various ways. For example, the reservoir may be formed by a rigid, semi-rigid or flexible material. In case a semi-rigid or flexible material is used, the reservoir may be collapsible or foldable, to make the irrigation system more compact prior to use. The reservoir is provided with an opening, closed by a lid 11, for filling the reservoir. Tubing 6, connecting the reservoir to the rest of the irrigation system, may be provided through the lid 11 or through other access points on the reservoir.

As one embodiment, the reservoir may be a collapsible reservoir of the type disclosed in US 2015/0335529, said document hereby being incorporated in its entirety by reference.

In order to render the irrigation system as portable as possible, the container preferably has a capacity of less than 5 liters, more preferred less than 3 liters and most preferred less than 2 liters. However, when the system is to be used for repeated irrigation, a larger capacity container may be necessary.

The catheter 2 is here embodied as a rectal catheter. The probe is provided with an inflatable retention member 23, such as an inflatable balloon, for fixing the catheter in a body cavity. The inflatable balloon preferably protrudes as a toroidal shape when inflated, and is essentially flush against the wall of the catheter when deflated. The inflatable retention member may also be referred to as a balloon, and is arranged close to the insertable tip 21, but at some distance from the end. Between the tip and the balloon, an opening, for dispensing liquid such as irrigation liquid to, or draining liquid from, the body, may be provided. The inflatable retention member may be made of any suitable material, such as PVC, latex, TPE or PU. However, other materials providing similar properties can likewise be used.

Further, the probe may be provided with a rearward enlarged part 22, providing an abutment to hinder too deep insertion. The catheter/probe is preferably provided with two lumens - one lumen for transfer of irrigation liquid through the probe, for discharge at the forward end, and one lumen for inflation and deflation of the balloon.

The probe may be of the type disclosed in WO 2014/154635, said document hereby being incorporated in its entirety by reference.

A control unit 3 may be provided. The control unit may be realized as a unitary, handheld unit, as in the illustrative example of Fig. 1. However, the control unit may alternatively be arranged as a base unit, e.g. arranged to be placed on the ground or floor during use.

The irrigation liquid can be any liquid which is capable of irrigating the body cavity of interest. In order to stimulate bowel movements, suitable irrigation liquids include water, hypertonic aqueous salt solutions, solutions or suspensions of cathartic agents, such as bisacodyl or phenolphthalein, and mineral oil.

Referring to Figs. 2 and 3, the container 1 may comprise a flexible side wall member 13, a rigid bottom portion 14, a rigid top portion 15, an inlet opening 11, and an outlet opening 16.

In the embodiments illustrated in Figs. 2 and 3, when assembled, the bottom portion 14 is arranged at a first open end of the side wall member 13 such that it seals the first open end. The rigid top portion 15 is arranged at a second open end of the side wall member 13 such that it seals the second open end.

In the embodiment of Fig. 2, both a filling opening 11 and an outlet opening 16 are provided in the rigid top portion. However, alternatively, as illustrated in Fig. 3, the outlet opening may instead be provided in the rigid bottom portion 14, which may e.g. be docked into a base portion 17. In such embodiments, the base portion 17 may comprise the controller 3, then realized as a base unit. The side wall 13 may be made from a flexible sheet material.

The control unit may be arranged connected to the container and the probe through tubing. Alternatively, the control unit may be directly connected to, and possibly even integrated with, the water container.

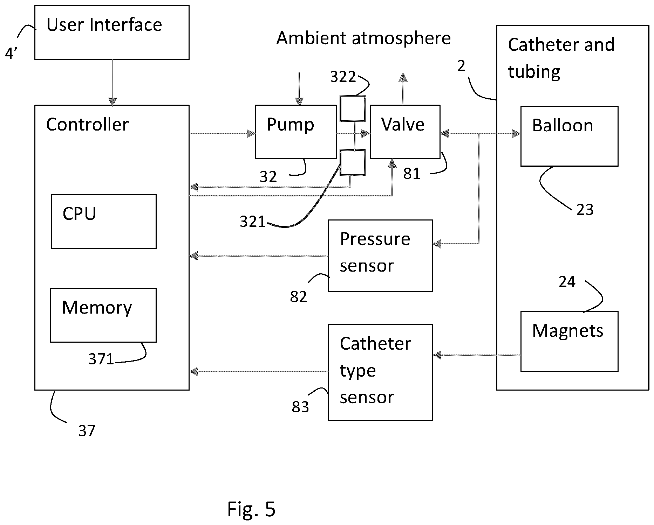

The first electrically operated pump 31, as well as the optional second electrically operated pump 32, for pumping irrigation liquid and inflation fluid, may be provided within the control unit 3, as the illustrative example of Figs. 4 and 5, but may also be arranged outside the bounds and housing of the control unit.

The control unit may comprise a display 33, and a user interface for providing input to the system, e.g. including one or several control elements. In the example of fig. 1, three control elements 34, 35 and 36 are provided. The control elements are preferably realized as depressible control buttons. The control unit is preferably waterproof. The control elements may thus be realized with thick pliable plastic or the like, designed to withstand many pushes.

However, alternatively, as in the illustrative example of Fig. 4, the control elements may instead be provided on a remote control 4, and may e.g. be realized as sensor areas on a touch screen.

The control elements 35 and 36 may here be used to activate the pump(s) for inflation/deflation of the inflatable retention member, transfer of irrigation liquid through the probe for irrigation (control element 36), releasing overpressure and/or draining the system of remaining liquid (control element 35). Separate control elements may also be provided for irrigation and inflation, so that inflation and deflation of the retention member may take place independently of the irrigation, and e.g. simultaneously.

The control unit or base unit comprises a controller 37. The control unit or base unit may further comprise one or more of the above-discussed pumps 31, 32 and a battery 38.

The controller 37 may be a microprocessor, MCU, comprising one or several central processing units, CPUs. The controller may also comprise two or more MCUs. However, the controller may also be realized in other ways, as is per se known in the art. Further, the controller 37 is preferably provided with one or several memories, either arranged integrated with the controller, or arranged as a separate component connected to the controller. The memory may be a ROM memory, such as an EPROM or EEPROM, or a RAM memory, such as Flash memory. However, many other types of memories may also be used, as is per se well known in the art.

Further, the controller is optionally connected to a wireless transceiver, which is adapted to transmit and receive data from the remote control 4, and/or other remote units. Hereby, the remote control may provide control data to the controller 37, for remote control of the control unit. Additionally or alternatively, the controller may transmit data about the irrigation procedure to the remote control or any other remote unit. The remote control 4 may e.g. be a smart phone or the like.

The battery 38 may be a rechargeable battery, chargeable through an external battery charger 5. Charging may e.g. occur through inductive charging from the charging station 5, or as direct charging from a connected electric conductor.

The evaluation and calibration as discussed in the foregoing are particularly useful in irrigation systems where the flow rate for the irrigation liquid and/or the size of an inflatable retention element are controlled based on calculations relating to the volume of air pumped per stroke. Such irrigation systems are e.g. known from European patent application no. 23202424.0 and European patent application no. 23202446.3, both by the same applicant and both hereby in their entirety incorporated by reference. Since the effective output volume may change over time, due to variations in the performance of the diaphragm pump, and also through leakage through valves, etc, the use of the present evaluation is highly useful for a calibration of the pump, or a measurement/estimation of the pump and subsequent adjustment of the calculations.

A method and system combining the calibration and evaluation as discussed in the foregoing with the improved inflation control as disclosed in European patent application no. 23202446.3 will now be discussed. The method/system is directed to a method and system for improved inflation control of an inflatable retention element, e.g. arranged on an insertable end of a rectal catheter or probe in case of a rectal or transanal irrigation system. The irrigation system comprises: a catheter having an inflatable retention member; an electrically operated pump for pumping a fluid into the catheter for inflation of the inflatable retention member; and an electrically operated valve for deflation of the inflatable retention member. A controller is provided for controlling the electrically operated pump and the electrically operated valve, the controller arranged to determine an inflation flow rate into the inflatable retention member from the electrically operated pump, and a deflation flow rate out from the inflatable retention member through the electrically operated valve, to continuously determine an inflation fluid volume based on the inflation flow rate and a deflation fluid volume based on the deflation flow rate, to continuously determine a present fluid volume in the inflatable retention member as the inflation fluid volume reduced by the deflation fluid volume, and to prohibit operation of the electrically operated pump when the present fluid volume is determined to have reached a volume safety threshold. Also here, the above-discussed evaluation and calibration enables use of such a control together with a diaphragm pump with increased precision and accuracy.

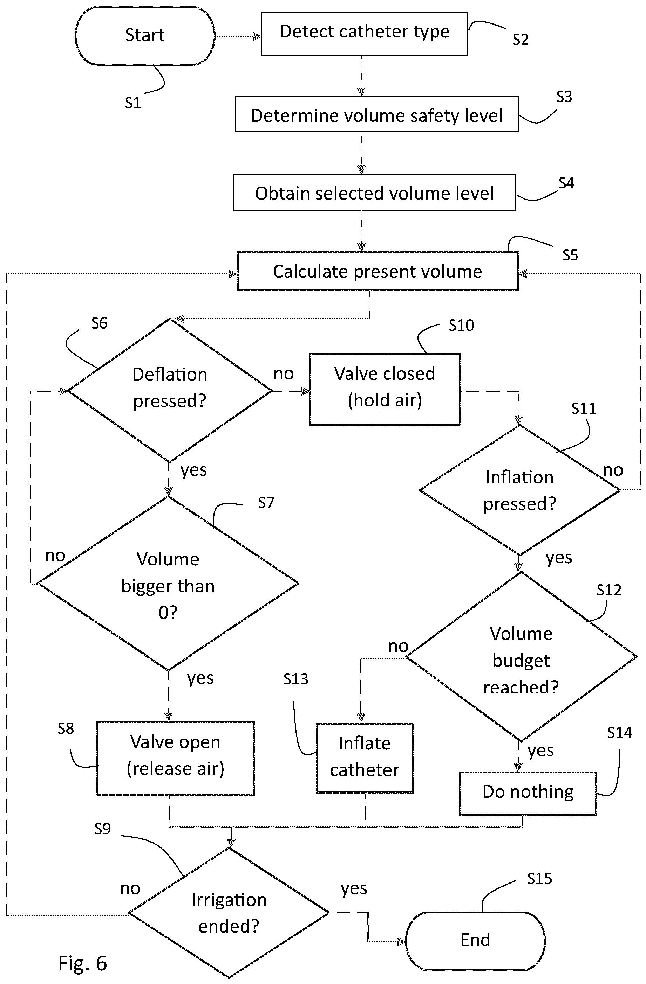

The part of the system handling the inflation and deflation of the inflatable retention member, and the operational procedure for handling inflation and deflation, will now be discussed in more detail with reference to Figs. 5 and 6.

A diaphragm pump 32 is here arranged to pump air through a conduit to the probe for inflation of the inflatable retention member 23. In the illustrated embodiment, different pumps may be used for irrigation and inflation. However, as already discussed, a single pump may be used for both purposes.

The pump 32 is controlled by the controller 37. Hereby, the controller may start and stop the pump, thereby starting and stopping the inflation, and may also possibly control the operating speed of the pump.

The pump 32 pumps air (or other inflation fluids) through an electrically controllable valve 81 and into the inflatable retention member 23. The valve 81 can be controlled by the controller 37 to assume a fully opened state and a fully closed state, and preferably also intermediate states. In an embodiment, the valve is continuously controllable between any state between the fully closed and opened states. When opened, the valve releases air from the inflatable retention member 23 into the ambient atmosphere, for deflation of the inflatable retention member.

A pressure sensor 82 is further arranged to measure the pressure in the balloon 23. The pressure sensor may be arranged inside the balloon, or in a conduit being in direct communication with the inflatable retention member. In the illustrative example, the pressure sensor is arranged in a conduit leading from the valve 81 to the balloon 23.

The pressure sensors may be any type of per se known pressure sensors. Preferably, the pressure sensor is a gauge pressure sensor, measuring the pressure relative to atmospheric pressure, and may e.g. be a piezoresistive sensor, such as a piezoresistive strain gauge, a capacitive sensor, an electromagnetic sensor, an optical sensor or the like. However, other types of pressure sensors may also be used.

The measurement output from the sensor 82 is forwarded to the controller 37, which may then control the operation of the pump 32 in accordance with this, as will be discussed in more detail in the following.

The controller 37 is arranged to determine an inflation flow rate into the inflatable retention member from the electrically operated pump 32, and a deflation flow rate out from the inflatable retention member through the electrically operated valve 81, to continuously determine an inflation fluid volume based on the inflation flow rate, a deflation fluid volume based on the deflation flow rate, and to continuously determine a present fluid volume in the inflatable retention member as the inflation fluid volume reduced by the deflation fluid volume.

The controller 37 may further control the electrically operated pump 32 and/or the electrically operated valve 81 based on the determined present fluid volume. For example, the controller 37 may be arranged to prohibit operation of the electrically operated pump when the present fluid volume is determined to have reached a volume safety threshold.

One and the same volume safety threshold may be used for all catheters useable with the irrigation system. However, in case various catheter types are used together with the irrigation system, different volume safety threshold may be determined for each of the catheter types. The catheter types may e.g. differ in material used or size of the catheter and/or the inflatable retention member, etc.

In case different volume safety thresholds are used for different catheter types, the controller of the irrigation system may be arranged to determine the catheter type automatically, and thereby automatically determining or choosing an appropriate volume safety threshold for this catheter type. To this end, the irrigation system may further comprise a property detection sensor 83, arranged to detect a property of the catheter, and wherein the controller is further arranged to set the volume safety threshold based on said property of the catheter. The catheter property may e.g. be catheter type and/or size.

In an embodiment, the property detection sensor 83 comprises a Hall sensor, arranged to determine the presence and/or configuration of magnets 24 in the catheter or in the tubing 6 connected to the catheter, the magnets being indicative of a size and or type of catheter. The Hall sensors may e.g. be arranged at, or in the vicinity, of where a catheter, or a tube connected to a catheter, is connected to another part of the system such as a base unit. The Hall sensor may be used simply to determine whether magnets are present or not. However, the Hall sensors may also be used to determine specific patterns or combinations of magnets, specific number of magnets, specific field strengths of the magnets, etc. Hereby, it is possible to distinguish between two, three, four, or even more, types or properties of catheters. Alternatively, detection of catheter property may occur in other ways, such as by provision of an RFID tag with information on the catheter or on the tubing connected to the catheter, and an RFID reader connected to the controller.

In an embodiment the controller may further be arranged to prohibit operation of the electrically operated pump when a pressure measured by the pressure sensor 82 is above a pressure threshold.

The irrigation system may further comprise a memory 371, e.g. integrated in the controller 37, storing a set of inflation levels, each level being correlated to a volume, and a user interface 4' arranged to receive input of a selected inflation level selected form said set of inflation levels. The controller 37 may then be arranged to operate the electrically operated pump until one of the selected inflation levels or the volume safety threshold has been reached. To this end, the controller 37 is hereby arranged to compare the present fluid volume to both the volume safety threshold level and the selected inflation level, and abort pumping as soon as any one of these levels has been reached. Preferably, the plurality of predetermined inflation levels comprises at least 3 different inflation levels, and preferably at least 5 different inflation levels. The size of the inflatable retention member generally corresponds to the fluid volume contained in the inflatable retention member.

The electrically operated pump may, whenever activated, always be operated at a constant speed, and e.g. supplied by a constant voltage and current. This makes it possible to determine, e.g. by simple experimental measurements, the air flow provided by the electrically operated pump at different balloon pressure levels.

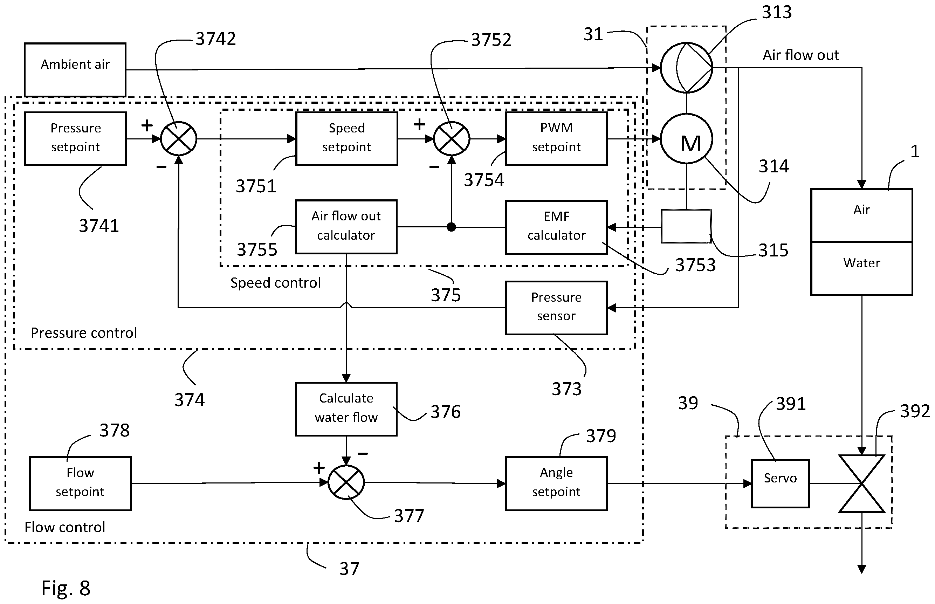

It has been found that when the pump is operated at a steady state, the air flow (L/min) decreases as the counter pressure (mbar), i.e. the pressure in the inflatable retention member, increases. Such a relationship for an exemplary catheter is illustrated in Fig. 8. The relationship generally follows an exponential graph with a decreasing form. Differently put, the air flow is initially, when the counterpressure is very low, at its highest, and then decreases rapidly as the counter pressure increases, and then less and less rapidly as the counter pressure continues to increase.

In an embodiment, the electrically operated pump is arranged to operate at a steady state whenever it is operated, i.e. by being supplied by a constant power. Hereby, the pump is generally operated intermittently at binary states, i.e. a steady state "on" state and an "off" state.

However, the pump need not be operated at a constant speed or with a steady state power. The voltage and the current may also be varied during operation of the pump. In such embodiments, the system may further comprise a power sensor for determining at least one of a current and a voltage supplied to the electrically operated pump. The controller may then be arranged to determine the inflation flow rate based on the current and/or voltage supplied to the electrically operated pump, based on input from the power sensor. The relationship between the airflow and the power, at various counter pressure levels, can again easily be determined experimentally.

The electrically operated valve may, whenever activated, always be fully opened. Thus, the electrically operated valve may be operated intermittently between a fully closed state and a fully opened state. This makes it possible to determine, e.g. by simple experimental measurements, the air flow provided through the electrically operated valve when in an open state at different balloon pressure levels.

In an embodiment, the electrically operated valve is arranged to always be in one of two states, viz. a fully opened state and a fully closed state. Hereby, the valve could be operated intermittently, to switch between the binary states.

However, the valve need not be operated only at two states, and one or more intermediate states, having varying degrees of openness, could also be contemplated. In one embodiment, the degree of openness of the valve may be continuously changed between a fully opened and a fully closed state. In such embodiments, the controller may be capable of determining the degree of openness of the valve, based e.g. on the control signals sent from the controller to the valve.

Alternatively, the system may comprise a sensor for determining the degree of openness of the valve. The controller may then be arranged to determine the deflation flow rate based on the degree of openness of the valve. The relationship between the airflow and the degree of openness, at various counter pressure levels, can again easily be determined experimentally.

Inflation of the inflatable retention member may be performed in various ways. In one embodiment, as illustrated in Fig. 6, an inflation process is started at S 1, e.g. by the user pushing a control element, such as a button or key.

In a subsequent step S2, the catheter type may be determined. This can, as discussed in the foregoing, e.g. be made by use of a catheter type sensor. However, in other embodiments this step may also be omitted.

Thereafter, in step S3, a volume safety level is determined. In some embodiments, there may be only one volume safety level, which is used for all catheters. This is particularly of relevance if the irrigation system only uses one type of catheter. In such embodiments, the only available volume safety level is determined to be used. However, if more than one type of catheter is available, such as catheters of different sizes, different volume safety levels may be used and assigned to different catheters. Determination of which volume safety level may then e.g. be made in dependence of the detected catheter type in step S2.

In step S4 a selected volume level may then be obtained, e.g. through input from a user interface.

All of the steps S2-S4 are optional, and one or more of these steps may be omitted.

In step S5, a present fluid volume is calculated, based on the pressure, the operation time of the electrically operated pump and valve, etc, as discussed in the foregoing.

It is then determined in step S6 whether a control element for deflation has been activated.

If activation of deflation control has been detected, it is determined in step S7 whether the present fluid volume is greater than zero, and if so, the valve is opened in step S8 for release of air from the inflatable retention element.

Alternatively, if it is determined that there is no activation of a deflation control, the valve is closed or maintained closed, step S10, and it is then determined whether a control element for inflation has been activated, step S11.

If activation of an inflation control has been detected, it is determined in step S12 whether the present fluid volume is greater than a volume budget, step S12. The volume budget is here considered to be the smallest of the determined volume safety level and the selected volume level. Alternatively, separate comparison steps for comparison with both the volume safety level and the selected volume level independently could also be made. In case the volume budget has not been reached, the catheter retention member is inflated, step S13, whereas if it is determined that the volume budget has been reached, the electrically operated pump is kept idle, step S14.

If it is determined in step S11 that there is no activation of any inflation control element, the process may return to step S5.

After steps S8, S13 and S14, respectively, the process may continue to step S9, where it is determined whether the irrigation procedure has been ended. If this is the case, the process ends, step S15, but otherwise, the process may return to step S5.

Hereby, the process may be repeated and iterated continuously until the irrigation procedure has been ended.

Notably, the sequence of the steps discussed in the foregoing may be different than the one discussed in relation to the exemplary embodiment of Fig. 6. For example, step S11 may be performed prior to step S6, step S12 may be performed earlier in the process, such as immediately following step S5, etc.

The calibration and evaluation discussed in the foregoing may additionally, or alternatively, be used with the method and system for improved control of a pumped irrigation liquid, such as the one disclosed in European patent application no. 23202424.0. The therein disclosed method/system is directed to a method and system for improved flow control of an irrigation liquid being pumped into the receiving site, such as the anal canal or rectum in case of a rectal or transanal irrigation system. The system comprises: a reservoir for an irrigating liquid; a pressure sensor for measuring the pressure in the reservoir; and a catheter for arrangement in a user; tubing providing a fluid communication path between said reservoir and said catheter. An electrically operated pump is provided for pumping air into the reservoir, thereby to indirectly pump irrigation liquid from the reservoir to the catheter through said tubing. Further, an electrically operable valve is operable to continuously control the degree of openness of said fluid communication path between a fully closed state and a fully opened state. A sensor is provided for estimation of power consumption or speed of the electrically operated pump and a controller is arranged to estimate an actual flow rate of irrigation liquid from the reservoir to the catheter, based on said estimation of the power consumption and/or speed, to obtain a desired flow rate from a user interface, and to continuously regulate the electrically operable valve based on the estimated actual flow rate, thereby adjusting the actual flow rate to said desired flow rate. Hereby, the actual flow rate can be estimated without any need for flow meters and the like. The above-discussed evaluation and calibration enables use of such a control together with a diaphragm pump with increased precision and accuracy.

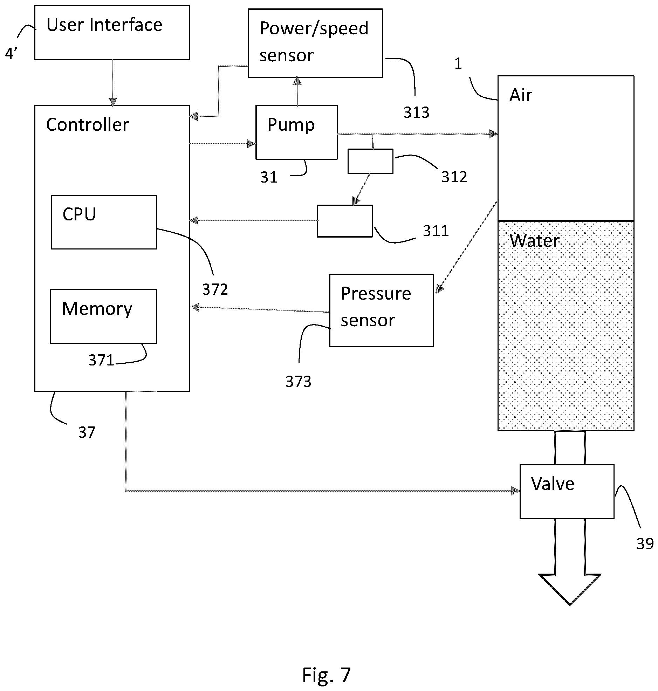

With reference to Fig. 7, a pump 31 is arranged to pump air through a conduit to the reservoir 1. Due to the air pumped into the reservoir, the pressure in the reservoir increases, thereby creating a flow of water out from the reservoir.

The conduit leading water out from the reservoir 1 passes an electrically operable valve 39.

A pressure sensor 373 is arranged to determine the pressure in the reservoir 1. Output from the pressure sensor is sent to the controller 37. The controller 37 may, as discussed in the foregoing, comprise a processor, such as a CPU 372, and a memory 371. The controller 37 communicates with a user interface 4' to receive input regarding e.g. a desired flow rate.

As for the previously discussed pressure sensors, this pressure sensor may also be any type of per se known pressure sensors. Preferably, the pressure sensor is a gauge pressure sensor, measuring the pressure relative to atmospheric pressure, and may e.g. be a piezoresistive sensor, such as a piezoresistive strain gauge, a capacitive sensor, an electromagnetic sensor, an optical sensor or the like. However, other types of pressure sensors may also be used. The pressure in the inflatable retention member can be directly determined by measuring by a pressure sensor. However, the pressure in the inflatable retention member may also be indirectly measured. For example, the pressure sensor may be a sensor measuring the load on the pump, from which it is possible to estimate the pressure in the reservoir.

The controller 37 controls the electrically operated pump 31 and the electrically operated valve 39, based on the determined pressure in the reservoir, as provided by the pressure sensor 373, a determined power consumption and/or speed of the pump, as provided by a power and/or speed sensor 313, and the desired flow rate, as received form the user interface 4'. The power sensor may e.g. be a current and/or voltage sensor, determining the current and/or voltage provided to the pump. The speed sensor may e.g. be a tachometer.

The controller 37 may be arranged to operate the pump so that an essentially constant pressure is provided and maintained in the reservoir. However, in other embodiments, the pump may instead be operated to provide a varying pressure in the reservoir. For such embodiments, the system may further be provided with a water level sensor or the like, arranged to provide an estimate of the current volume of water in the reservoir to the controller.

The controller 37 is arranged to estimate an actual flow rate of irrigation liquid from the reservoir to the catheter based on at least the estimation of the power consumption and/or speed and to continuously regulate the electrically operable valve based on the estimated actual flow rate, thereby adjusting the actual flow rate to the desired flow rate.

A more detailed exemplary realization of the system of Fig. 7 will now be discussed with reference to Fig. 8.

As illustrated in Fig. 6, the controller may comprise a pressure control 374, which in turn may comprise a speed control 375.

In the pressure control 374 the pressure in the reservoir 1 is determined by the pressure sensor 373, and this determined pressure is used in conjunction with a desired pressure, a pressure setpoint 3741, in regulation unit 3742 to provide a pressure control signal to control the operation of the pump 31 through the speed control 375. In this embodiment, the electrically operated pump comprises a motor 314 driving a mechanical pump 313. The pressure level determined by the pressure setpoint may be a fixed pressure level, which may be a predetermined level, for embodiments where the system is arranged to maintain a constant pressure in the reservoir.

The speed control 375 receives the pressure control signal and determines a corresponding desired speed for the pump motor 312 in a speed setpoint 3751. The desired speed is forwarded to a regulation unit 3752.

One or more power consumption and/or speed sensor 315 may be used to determine the power consumption and/or speed of the motor. The pump motor may e.g. be a brushed DC motor, and the power consumption sensor may e.g. comprise a voltage difference sensor and a current sensor.

The output from the sensor(s) 315 may be forwarded to an EMF calculator 3753, estimating an electromagnetic force (EMF) and/or speed of the pump motor 312. The output from the EMF calculator 3753 may also be forwarded to the regulation unit 3752.

The regulation unit 3752 may use the desired speed from the speed setpoint 3751 in conjunction with the estimated power consumption and/or speed from the EMF calculator 3753 to determine speed control signal. This signal is forwarded to a PWM setpoint 3754, determining a pulse width modulation (PWM) for driving the pump motor 314.

The power consumption and/or speed signal provided by the EMF calculator 3753 is further forwarded to an air flow out calculator 3755, which uses this to provide an estimate of the flow rate and/or volume of air pumped by the electrically operated pump 31.

The estimate provided by the air flow out calculator 3755 is forwarded to a unit 376 calculating a nominal output flow rate of water from the reservoir based on this estimated pumped air flow and/or volume. The nominal output flow rate of water corresponds to a flow rate that will occur when the valve is in a fully opened state, i.e. a maximum output flow rate, where there are no restrictions in the water outflow.

A further unit, a flow setpoint 378, determines a desired flow rate, e.g. based on input from a user interface. The desired flow rate and the estimated nominal output flow rate are used in conjunction by regulation unit 377 to provide a valve control signal.

As discussed in the foregoing, the valve may be of the type disclosed in US 2006/0114148. The electrically operable valve may comprise a movable arm that is connected to a constriction structure, said constriction structure being arranged opposite to an abutment, and with the tube forwarding the irrigation liquid from the reservoir to the catheter extending between the constriction structure and the abutment. Movement of the moveable arm moves the constriction structure to control compression of the tube between the constriction structure and the abutment. The valve can be controlled by the controller 37 to assume a fully opened state and a fully closed state, and preferably also intermediate states. In an embodiment, the valve is continuously controllable between any state between the fully closed and opened states. The mechanical valve 392 may comprise a moveable arm which may be movable by rotation, and the rotation may be effected by a servo motor 391. The angular position of the moveable arm will consequently determine the degree of openness of the valve.

In the illustrative example, the valve control signal from the regulation unit 377 may be forwarded to an angle setpoint 379, where an angle for the moveable arm in the valve 39 is determined based on the valve control signal and forwarded to the servo 391 to move the movable arm into this angular position.

In use, the reservoir is filled with a desired volume of irrigation liquid. The pump is then operated to prime the tank and increase the pressure to a target level. The system is then used for irrigation. Hereby, the pump may be continuously operated to maintain the pressure in the reservoir constant, when the electrically operated valve is opened to release irrigation liquid out from the reservoir. The flow rate of the irrigation liquid out from the reservoir is estimated based on the power consumption and/or speed of the pump and is used to control the electrically operated valve to obtain a desired output flow rate.

The person skilled in the art realizes that the present invention by no means is limited to the preferred embodiments described above. On the contrary, many modifications and variations are possible within the scope of the appended claims. For example, evaluation or calibration of the diaphragm pump may be used for a pump for pumping irrigation liquid, or for inflation of an inflatable retention member, or both. The evaluation/calibration could also be used for diaphragm pumps used for different pumping actions in an irrigation system, or also for other types of systems. Further, the diaphragm pump(s) is preferably used for pumping air, but may also be used for pumping other gases or fluids. The irrigation system could also be used for other purposes than rectal or transanal irrigation, such as other forms of irrigation, or for other medical procedures. Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements. Further, a single unit may perform the functions of several means recited in the claims.

## Claims

1. A method for evaluation of the operation of a diaphragm pump for pumping air in an irrigation system, the method comprising:

   operating the diaphragm pump;
   measuring pressures at two opposite end positions for a diaphragm of the diaphragm pump in at least two different pump cycles of the diaphragm pump;
   estimating a current effective output volume per pumping cycle for the diaphragm pump based on the measured pressures;
   comparing the estimated current effective output volume to a previously determined, primary effective output volume; and
   provide an evaluation of the operation of the diaphragm pump.

2. The method of claim 1, further comprising direct or indirect measurement of an operational speed of the diaphragm pump and estimation of at least one of a pump flow or total pumped volume based on said operational speed and said primary effective output volume.

3. The method of claim 2, wherein the operational speed is directly measured by a sensor associated with the diaphragm pump.

4. The method of claim 2, wherein the operational speed is indirectly measured by measuring at least one of an operational voltage and current provided to the diaphragm pump.

5. The method of any one of the preceding claims, wherein the diaphragm pump, during the step of measuring said pressure s, is operated to pump air into a closed reference volume having a known total volume.

6. The method of claim 5, wherein estimating a current effective output volume per pumping cycle for the diaphragm pump comprises determination based on the measured pressures at the end position where the diaphragm is in a fully retracted position corresponding to a maximum volume and at the end position where the diaphragm is in a fully extended position corresponding to a minimum volume.

7. The method of claim 6, wherein the current effective output volume per pumping cycle is determined based on a subtraction of the minimum volume from the maximum volume.

8. The method of any one of the preceding claims, wherein the provision of the evaluation of the operation of the diaphragm pump comprises determining whether a difference between the current effective output volume and the primary effective output volume exceeds a first threshold, and if this is the case assigning a new value to the primary effective output based on the estimation of the current effective output volume.

9. The method of any one of the preceding claims, wherein the provision of the evaluation of the operation of the diaphragm pump comprises determining of whether a difference between the current effective output volume and the primary effective output volume exceeds a second threshold, and if this is the case adjust at least one operational parameter of the diaphragm pump to decrease the difference between the current effective output volume and the primary effective output volume.

10. The method of any one of the preceding claims, wherein the provision of the evaluation of the operation of the diaphragm pump comprises determining of whether a difference between the current effective output volume and the primary effective output volume exceeds a third threshold, and if this is the case issue an alarm signal and/or make the diaphragm pump inoperable.

11. An irrigation system for medical use comprising:

   a catheter;
   an irrigation liquid reservoir;
   an electrically operated diaphragm pump for pumping air into the irrigation liquid reservoir and/or into the catheter ;
   a pressure sensor associated with an output end of the diaphragm pump; and
   a controller for evaluation of the operation of the diaphragm pump, the controller being arranged to:

      measuring the pressure at two opposite end positions for a diaphragm of the diaphragm pump in at least two different pump cycles of the diaphragm pump during operation of the diaphragm pump;
      estimating a current effective output volume per pumping cycle for the diaphragm pump based on the measured pressures;
      comparing the estimated current effective output volume to a previously determined, primary effective output volume; and
      provide an evaluation of the operation of the diaphragm pump.

12. The irrigation system of claim 11, wherein the electrically operated diaphragm pump is arranged to pump air into the irrigation liquid reservoir for indirect pumping of irrigation liquid from the irrigation liquid reservoir to the catheter.

13. The irrigation system of claim 11 or 12, wherein the catheter has an inflatable retention member and wherein the electrically operated diaphragm pump is arranged to pump air into the catheter for inflation of the inflatable retention member.

14. The irrigation system of any one of the claims 11-13, further comprising at least one of a current or voltage meter for indirect measurement of an operational speed of the diaphragm pump or a sensor associated with the diaphragm pump for direct measurement of the operation speed of the diaphragm pump.

15. The irrigation system of any one of the claims 11-14, further comprising a closed reference volume having a known total volume.

16. The irrigation system of claim 15, wherein the closed reference volume is at least partly formed by a cavity arranged within a housing which also accommodates the diaphragm pump and fluidly connected to a fluid path extending between the diaphragm pump and the catheter.

17. The irrigation system of any one of the claims 11-16, wherein the catheter is a rectal catheter, and wherein the catheter system is a rectal, anal or transanal irrigation system.

Fig. 1

Fig. 2

Fig. 3

Fig. 4

User Interface

4'

Ambient atmosphere

Catheter and tubing

2

Controller

322

Pump

32

Valve

81

Balloon

23

CPU

321

Pressure sensor

82

Memory

371

Catheter type sensor

83

37

24

Magnets

Fig. 5

Start — S1

Detect catheter type — S2

Determine volume safety level — S3

Obtain selected volume level — S4

Calculate present volume — S5

S6 — Deflation pressed? — no → S10 — Valve closed (hold air)

S10 → S11 — Inflation pressed? — no

yes ↓

S7 — Volume bigger than 0?

no

S11 — yes ↓

S12 — Volume budget reached?

no →

S13 — Inflate catheter

yes ↓

S7 — yes ↓

S8 — Valve open (release air)

S14 — Do nothing

S9 — Irrigation ended? — no / yes → S15 — End

Fig. 6

Fig. 7

Fig. 8

EP 4 785 974 A1

Europäisches
Patentamt

European
Patent Office

Office européen
des brevets

# EUROPEAN SEARCH REPORT

**Application Number**

EP 25 15 5193

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 4 383 112 A2 (DEKA PRODUCTS LP [US]) 12 June 2024 (2024-06-12) | 1-16 | INV. A61M3/02 A61M25/10 |
| A | * paragraph [0052] * | 17 | |
| | ----- | | |
| Y | US 2024/209847 A1 (ARNOLD MATTHIAS [DE]) 27 June 2024 (2024-06-27) | 1-16 | |
| A | * claims * | 17 | |
| | ----- | | |

**TECHNICAL FIELDS SEARCHED (IPC)**

A61M

The present search report has been drawn up for all claims

| Place of search | Date of completion of the search | Examiner |
|---|---|---|
| The Hague | 16 June 2025 | Vadot, Pierre |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 25 15 5193

This annex lists the patent family members relating to the patent documents cited in  the above-mentioned European search report.
The members are as contained in the European Patent Office EDP file on
The European Patent Office is in no way liable for these particulars which are merely given for the purpose of information.

16-06-2025

| Patent document cited in search report | | Publication date | Patent family member(s) | | Publication date |
|---|---|---|---|---|---|
| EP 4383112 | A2 | 12-06-2024 | CA | 2950325 A1 | 03-12-2015 |
| | | | EP | 3148607 A2 | 05-04-2017 |
| | | | EP | 3578212 A1 | 11-12-2019 |
| | | | EP | 3736721 A1 | 11-11-2020 |
| | | | EP | 4383112 A2 | 12-06-2024 |
| | | | MX | 385331 B | 18-03-2025 |
| | | | WO | 2015183981 A2 | 03-12-2015 |
| US 2024209847 | A1 | 27-06-2024 | CN | 114127419 A | 01-03-2022 |
| | | | DK | 3997339 T3 | 02-12-2024 |
| | | | EP | 3997339 A1 | 18-05-2022 |
| | | | JP | 2022539428 A | 08-09-2022 |
| | | | US | 2024209847 A1 | 27-06-2024 |
| | | | WO | 2021008788 A1 | 21-01-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160193403 A
- WO 23227571 A
- EP 3338827 A
- EP 23202424
- EP 23202446
- US 20060114148 A
- US 20150335529 A
- WO 2014154635 A